(19) Europäisches Patentamt
European Patent Office
Office européen des brevets

(11) Publication number: 0 163 258
A2

(12) # EUROPEAN PATENT APPLICATION

(21) Application number: 85106333.9

(22) Date of filing: 23.05.85

(51) Int. Cl.⁴: A 61 K 31/48

(30) Priority: 30.05.84 GB 8413795

(43) Date of publication of application:
04.12.85 Bulletin 85/49

(84) Designated Contracting States:
AT BE CH DE FR GB IT LI LU NL SE

(71) Applicant: SANDOZ AG
Lichtstrasse 35
CH-4002 Basel(CH)

(84) Designated Contracting States:
BE CH FR GB IT LI LU NL SE

(71) Applicant: SANDOZ-PATENT-GMBH
Humboldtstrasse 3
D-7850 Lörrach(DE)

(84) Designated Contracting States:
DE

(71) Applicant: SANDOZ-ERFINDUNGEN
Verwaltungsgesellschaft m.b.H.
Brunner Strasse 59
A-1235 Wien(AT)

(84) Designated Contracting States:
AT

(72) Inventor: Steven, Ian, D.
45 North East Road
Collinswood, S.A. 5081(AU)

(54) **New use of dihydroergotamine.**

(57) Dihydreorgotamine for the treatment of Infectious Mononucleosis.

EP 0 163 258 A2

0163258

Case 118-6350

NEW USE OF DIHYDROERGOTAMINE

The present invention relates to a new pharmaceutical use of dihydroergotamine.

Dihydroergotamine, of formula I

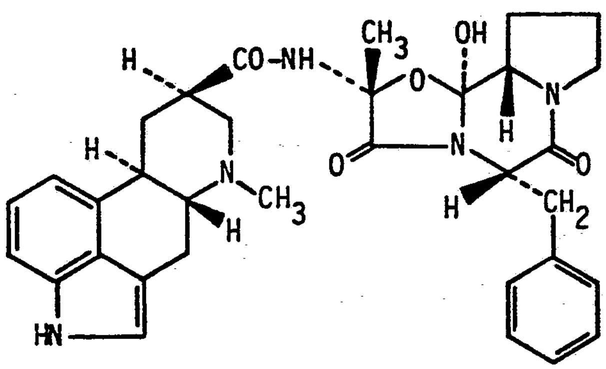

(I)

is the 9,10 dihydro derivative of ergotamine and may be prepared e.g. by catalytic hydrogenation of the natural alkaloid or by total synthesis.

The compound is well known from the literature and its activity profile has been established from data accumulated over many years in clinical trials and animal tests.

Dihydroergotamine is indicated to be useful against hypotensive and orthostatic circulatory disturbances, for the treatment of acute attacks of migraine and related vascular headaches and the interval treatment of migraine and other vascular headaches.

The compound may be administered in free base form or in pharmaceutically acceptable acid addition salt form. Such acid addition salt forms are known, exhibit the same order of activity as the free base forms and are readily prepared in conventional manner. Representative acid addition salt forms include the methanesulfonate.

Furthermore dihydroergotamine is possibly effective in Herpes Zoster [Steven I.D., letter to editor, Modern Medicine 9, 88 (1982)], Herpes Simplex [Steven I.D., letter to editor, Australian Family Physician, 8, 560 (1983)] and in the treatment of mumps [e.g. Vaidya R.S., Antiseptic 54, 605 (1957)].

It has now been surprisingly found that dihydroergotamine is useful in the treatment of Infectious Mononucleosis.

Infectious Mononucleosis, also called glandular fever, is a viral infection of significant and often long lasting morbidity. [See for example Evans A.S. et al., Epstein-Barr Virus in Evans A.S. ed. Viral Infections of Humans, 2nd Edition Plenum Medical, New York and London, 270 (1982)]. Patients who are significantly unwell generally take some weeks to recover enough to resume normal activity. General malaise frequently occurs for long periods after acute symptoms have resolved.

The efficacy of dihydroergotamine in the treatment of Infectious Mononucleosis was established in clinical trials with patients suffering from clinically diagnosed Infectious Mononucleosis, including the following:

Fifteen patients with clinical Infectious Mononucleosis plus confirmed haematological evidence (the presence of a lymphocytosis with at least 50 % lymphocytes and 10 % atypical lymphocytes) and heterophile antibodies (Evans A.S. et al. cited above) being present, were admitted to this trial.

Dihydroergotamine was administered subcutaneously at a dosage of 0.5 mg daily during 5 days. One case clinically recovered with 2 doses and one case with 3 doses. No case in the trial was given more than 5 doses. One pre-pubertal boy was treated and the dosage was proportionately decreased to 0.35 mg. The results are outlined in the following table:

| CASE NO. | SEX | AGE | TIME FROM ONSET OF SYMPTOMS TO START OF TREATMENT (DAYS) | RETURN TO NORMAL OCCUPATION (DAYS) |
|---|---|---|---|---|
| 1 | M | 10 | 9 | 5 |
| 2 | M | 17 | 7 | 5 |
| 3 | M | 19 | 14 | 5 |
| 4 | F | 18 | 5 | 6 |
| 5 | F | 17 | 15 | 4 |
| 6 | F | 18 | 5 | 11 |
| 7 | F | 14 | 15 | 4 |
| 8 | M | 18 | 4 | 6 |
| 9 | M | 13 | 10 | 9 |
| 10 | F | 15 | 11 | 9 |
| 11 | F | 14 | 5 | 10 |
| 12 | M | 53 | 26 | 5 |
| 13 | F | 18 | 8 | 7 |
| 14 | M | 23 | 4 | 5 |
| 15 | M | 19 | 10 | 14 |

The compound was well tolerated by the patients. Patients generally reported that they returned to completely normal activity about 1 week after they resumed their normal occupation. General malaise was in general not a feature of their illness.

Dihydroergotamine is therefore useful as an agent against Infectious Mononucleosis.

In accordance with the foregoing the present invention provides a method for the treatment of Infectious Mononucleosis which comprises administering a therapeutically effective amount of dihydroergotamine or a pharmaceutically acceptable acid addition salt thereof to a subject in need of such treatment.

For this use, the dosage used will naturally depend on the mode of administration used and treatment desired. However, in general, satisfactory daily dosages are from about 0.1 to about 20 mg, and suitable dosage forms comprise from about 0.05 mg to about 10 mg of the compound admixed with a solid or liquid pharmaceutical carrier or diluent. Preferably the compound is administered parenterally, e.g. subcutaneously.

Preferably the compound is administered at a daily dose of from 0.25 to 5 mg, e.g. 0.5 mg.

The compound may be administered in free base form or pharmaceutically acceptable salt form, e.g. the methanesulfonate form.

Conveniently the compound is administered in the form of a pharmaceutical composition comprising the compound in free base form or in pharmaceutically acceptable acid addition salt form in association with a pharmaceutical carrier or diluent.

The known pharmaceutical compositions are suitable and may be made by conventional techniques to be in the form of capsules, tablets, suppositories, dispersible powders, syrups, elixirs, suspensions or solutions as appropriate for enteral or parenteral administration. Preferably unit dosage forms are used. The compositions may contain conventional pharmaceutical excipients, e.g. diluents and carriers, such as water, alcohols, natural or hardened oil and waxes, calcium and sodium carbonate, calcium phosphate, kaolin, talc and lactose. Other excipients which may be used include suspending agents, lubricating agents, disintegrating agents, etc.

Compositions in tablet form may be coated by conventional techniques to delay disintegration of the tablet and/or absorption of the active ingredient in the gastro-intestinal tract and thereby provide sustained action over a long period.

Pharmaceutical compositions comprising dihydroergotamine for use in the method of the present invention are new. In a further aspect the present invention accordingly also provides:

a) the use of dihydroergotamine in free base form or in pharmaceutically acceptable acid addition salt form, for the manufacture of a pharmaceutical composition for the treatment of Infectious Mononucleosis;

b) a pharmaceutical composition which incorporates dihydroergotamine in free base form or in pharmaceutically acceptable acid addition salt form as an active agent, for use in the treatment of Infectious Mononucleosis;

c) a pack or dispenser device containing dihydroergotamine in free base form or in pharmaceutically acceptable acid addition salt form, for use in the treatment of Infectious Mononucleosis. The pack or dispenser device may contain for example a plurality of unit dosage forms containing the substance. These may be packed in metal or plastic foil, e.g. as in a blister pack. The pack or dispenser device may be together with instructions for administration of the compound.

The following is illustrative of suitable compositions for administration of the compound for the treatment of Infectious Mononucleosis. Other compositions which are commercially available may be used.

1. Capsules

| Ingredient | Weight (mg) |
|---|---|
| Dihydroergotamine methanesulfonate | 1 |
| Lactose (200 mesh/inch) | 133.5 |
| Corn starch | 92 |
| Silicion dioxide (Aerosil 200) | 1.2 |
| Magnesium stearate | 2.3 |
| Total | 230 |

The ingredients are compounded in conventional manner and filled into a 62 mg capsule.

2. Ampoules

| Ingredient | Weight/vol. (mg/ml) |
|---|---|
| Dihydroergotamine methanesulfonate | 2.5 |
| Sodium chloride | 8 |
| Water for injectable solutions | up to 1 ml |

Ampoules are filled with 1 ml of the solution, sealed and sterilized at 121 ° C for 15 minutes.

WHAT WE CLAIM IS:

1. A method for the treatment of Infectious Mononucleosis which comprises administering a therapeutically effective amount of dihydroergotamine or pharmaceutically acceptable acid addition salt thereof to a subject in need of such treatment.

2. The use of dihydroergotamine in free base form or in pharmaceutically acceptable acid addition salt form, for the manufacture of a pharmaceutical composition for the treatment of Infectious Mononucleosis.

3. A pharmaceutical composition which incorporates dihydroergotamine in free base form or in pharmaceutically acceptable acid addition salt form as an active agent, for use in the treatment of Infectious Mononucleosis.

4. A pack or dispenser device containing dihydroergotamine in free base form or in pharmaceutically acceptable acid addition salt form, for use in the treatment of Infectious Mononucleosis.